(19) Europäisches Patentamt
European Patent Office
Office européen des brevets

(11) EP 0 345 598 B1

(12) EUROPÄISCHE PATENTSCHRIFT

(45) Veröffentlichungstag und Bekanntmachung des Hinweises auf die Patenterteilung:
**12.08.1998 Patentblatt 1998/33**

(51) Int Cl.6: **C07H 15/252**, C07H 15/26, A61K 31/70

(21) Anmeldenummer: **89109689.3**

(22) Anmeldetag: **30.05.1989**

# (54) Zytostatisch wirksame Anthracyclinderivate

Anthracycline derivatives with a cytostatic activity

Dérivés d'anthracyclines à activité cytostatique

(84) Benannte Vertragsstaaten:
**AT BE CH DE ES FR GB GR IT LI LU NL SE**

(30) Priorität: **04.06.1988 DE 3819092**

(43) Veröffentlichungstag der Anmeldung:
**13.12.1989 Patentblatt 1989/50**

(73) Patentinhaber: **BEHRINGWERKE Akfiengesellschaft**
**35001 Marburg (DE)**

(72) Erfinder:
- **Hermentin, Peter, Dr.**
  **D-3550 Marburg (DE)**
- **Raab, Ernst**
  **D-3550 Marburg-Dagobertshausen (DE)**
- **Kolar, Cenek, Dr.**
  **D-3550 Marburg (DE)**
- **Gerken, Manfred, Dr.**
  **D-3550 Marburg (DE)**
- **Hoffmann, Dieter, Dr.**
  **D-3551 Lahntal (DE)**
- **Kraemer, Hans Peter, Dr. Dr.**
  **D-3550 Marburg (DE)**
- **Stache, Ulrich, Dr.**
  **D-6238 Hofheim am Taunus (DE)**

(74) Vertreter: **Then, Johann et al**
**Hoechst AG**
**Patent- und Lizenzabteilung**
**Gebäude K 801**
**65926 Frankfurt am Main (DE)**

(56) Entgegenhaltungen:
**EP-A- 0 063 776** **EP-A- 0 270 992**
**EP-A- 0 290 774**

- **CHEMICAL ABSTRACTS, Band 92, Nr. 17, 28. April 1980, Seite 631, Zusammenfassung, Nr. 147142f, Columbus, Ohio, US; & JP-A-54 141 759 (MICROBIOCHEMICAL RESEARCH FOUNDATION) 05-11-1979**
- **J. MED. CHEM., Band 29, 1986, Seiten 2120-2122, American Chemical Society; E.M. ACTON et al.: "N-(2-Hydroxyethyl)doxorubicin from Hydrolysis of 3'-Deamino-3'-(3-cyano-4-morpholinyl)doxorubicin"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 345 598 B1

Printed by Jouve, 75001 PARIS (FR)

**Beschreibung**

Die Erfindung betrifft neue, zytostatisch wirksame Anthracyclinderivate der allgemeinen Formel I, Verfahren zu ihrer Herstellung sowie ihre Verwendung als Arzneimittel.

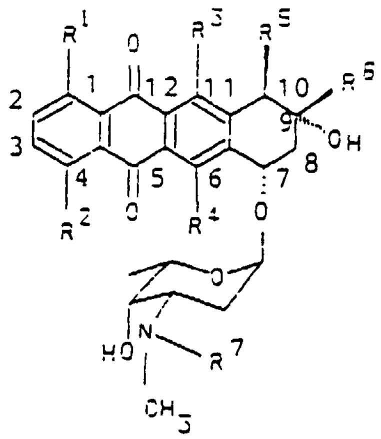

I

Für Formel I gilt:

$R^1$ ist Wasserstoff oder eine Hydroxygruppe,
$R^2$ ist Wasserstoff, eine Hydroxy- oder eine Methoxygruppe,
$R^3$ ist Wasserstoff oder eine Hydroxygruppe,
$R^4$ ist Wasserstoff oder eine Hydroxygruppe,
$R^5$ ist Wasserstoff, eine Hydroxy- oder eine Methoxycarbonylgruppe,
$R^6$ ist $CH_2CH_3$, $COCH_3$, $COCH_2OH$, $CHOHCH_3$ oder $CHOHCH_2OH$ und
$R^7$ ist ein organischer Substituent mit 2 bis 6 C-Atomen, welcher mindestens ein Sauerstoff- oder Stickstoff- oder Schwefelatom oder eine C-C Doppelbindung oder eine C-C Dreifachbindung enthält, wobei die Doppelbindung Bestandteil eines heteroaromatischen System sein kann und das Sauerstoff-, Stickstoff- oder Schwefelatom Bestandteil eines heterocyclischen Systems ist.
$R^7$ ist bevorzugt

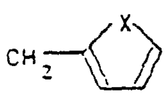 oder 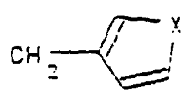

mit X = O, N oder S, wobei der Heterocyclus gegebenenfalls durch $-CH_3$, $-NO_2$, $-CH_2OH$, -Cl oder -Br substituiert sein kann, bevorzugt jedoch nicht substituiert ist.
R7 ist bevorzugt ein 2-Picolyl- (2-Methylen-pyridyl), 3-Picolyl- oder 4-Picolyl-Rest, substituiert oder unsubstituiert, besonders bevorzugt jedoch ein 2-Picolyl- oder 4-Picolyl-Rest,
$R^7$ ist bevorzugt ein Allyl-, Crotyl- oder Propargyl-, besonders bevorzugt ein Allyl-Rest, oder
$R^7$ ist bevorzugt Glycidyl.

Die Verbindung der Formel I kann gegebenenfalls auch als Säureadditionssalz von physiologisch unbedenklichen anorganischen oder organischen Säuren vorliegen.

Es ist bekannt, daß eine Vielzahl von Anthracyclinen zytostatische Wirksamkeit besitzen und daß einige Anthracycline, wie z. B. Adriamycin, Daunomycin, Aclacinomycin, 4'-epi-Adriamycin, 4'-Methoxyadriamycin oder 4'-Desoxyadriamycin für die Therapie von Tumoren verwendet werden.

Bei der tumortherapeutischen Verwendung dieser bekannten Anthracycline liegt ein wesentliches Problem darin, daß sie neben der gewünschten zytostatischen Wirksamkeit unerwünschte Nebenwirkungen aufweisen, wie beispielsweise eine hämatologische oder cardiale Toxizität.

Ausgehend von diesem Stand der Technik ist es Aufgabe der vorliegenden Erfindung, neue Anthracyclinderivate zu schaffen, die möglichst nicht kreuzresistent gegenüber Adriamycin sind und die sich durch ein neues Wirkungsspektrum und eine im Vergleich zu Adriamycin geringere Toxizität auszeichnen und somit vorteilhafte Anwendung in

der Tumortherapie finden können.

Zu diesem Zweck wurde bereits vorgeschlagen, Rhodosaminyl-Anthracyclinone auf photolytischem Wege zu monodemethylieren und die so gewonnenen 3'-N-Methyldaunosaminyl-anthracyclinone an ihrer Methylamino-Funktion selektiv zu modifizieren bzw. zu substituieren, wodurch zahlreiche neue zytostatisch wirksame Anthracycline erhalten werden.

Beispielsweise wurde bereits vorgeschlagen, 3'-N-Methyl-daunosaminyl-anthracyclinone so zu derivatisieren, daß $R^7$ in Formel I Cyanomethyl, COR* oder $CH_2R^{10}$ ist, wobei R* = H, $CH_3$, $CF_3$ oder $CCl_3$ und $R^{10}$ $C_1$- bis $C_8$-Alkyl, substituiertes Alkyl, Phenyl oder substituiertes Phenyl darstellen kann, wobei der Phenylring in ortho-, meta- oder para-Stellung durch Methyl, Ethyl, Hydroxy, Methoxy, Ethoxy, Nitro, Cyano, Fluor, Chlor oder Brom substituiert sein kann.

Aus E. M. Acton et al., J. Med. Chem. 29, 2120 (1986) sind ebenfalls Anthracyclinderivate bekannt, von den Verbindungen der gemäß Art. 54 (3) EPÜ zu nennenden EP-A-0 270 992 sind die in der vorliegenden Anmeldung beanspruchten Verbindungen abgegrenzt.

Es wurden nun die Anthracyclinderivate der Formel I gefunden, die gegenüber den Anthracyclinen des Standes der Technik in vitro nicht kreuzresistent sind und bezüglich ihrer Wasserlöslichkeit und/oder Reaktivität und/oder Toxizität Vorteile aufweisen. Diese Verbindungen sind charakterisiert durch Formel I.

So läßt sich z.B. die für die Applizierbarkeit wichtige Wasserlöslichkeit der bereits vorgeschlagenen Benzylderivate ($R^7$ = Benzyl oder substituiertes Benzyl) verbessern, wenn der Phenyl-Kern der Benzylgruppe ein Stickstoffatom enthält, also durch einen Pyridyl-Rest ersetzt ist.

Überraschenderweise wurde sowohl die Wasserlöslichkeit als auch die zytostatische Aktivität einer Verbindung der Formel I im Vergleich zur 3'-N-Benzylverbindung günstig beeinflußt, wenn $R^7$ Furfuryl oder Thenyl ist.

Völlig überraschend wurde gefunden, daß, wenn $R^7$ Glycidyl ist, die Zytotoxizität einer Verbindung der Formel I drastisch erhöht ist, so daß dieser Substituent einen besonderen Vorteil aufweist.

Wenn $R^7$ Allyl ist, wird besonders gute zytostatische Aktivität des Anthracyclinderivats beobachtet.

Ein Verfahren zur Herstellung der neuen erfindungsgemäßen zytostatisch wirksamen Anthracyclinderivate besteht darin, daß man eine Verbindung der Formel I, in welcher $R^1$ = H oder OH, $R^2$ = H, OH oder $OCH_3$, $R^3$ = H oder OH, $R^4$ = H oder OH, $R^5$ = H, OH oder $COOCH_3$, $R^6$ = $CH_2CH_3$, $COCH_3$, $COCH_2OH$, $CHOHCH_3$ oder $CHOHCH_2OH$ und $R^7$ = H ist, entweder in an sich bekannter Weise (Tong et al., J. Med. Chem. (1979) 22, 912) in Gegenwart von Natriumcyanoborhydrid mit einem Aldehyd von 2 bis 6 C-Atomen, welcher mindestens ein Sauerstoff-, Stickstoff- oder Schwefelatom oder eine C-C Doppelbindung oder eine C-C Dreifachbindung enthält,
wobei die Doppelbindung auch Bestandteil eines heteroaromatischen Systems sein kann und das Sauerstoff-, Stickstoff- oder Schwefelatom Bestandteil eines heterocyclischen Systems ist,
oder in an sich bekannter Weise unter wasserfreien Bedingungen in Gegenwart einer Base mit einer Organo-Halogenverbindung von 2 bis 6 C-Atomen, welche mindestens ein Sauerstoff-, Stickstoff- oder Schwefelatom oder eine C-C Doppelbindung oder eine C-C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist,
zu einer Verbindung der Formel I umsetzt, in welcher $R^1$ bis $R^6$ die angegebene Bedeutung haben und $R^7$ ein organischer Substituent mit 2 bis 6 C-Atomen ist, welcher mindestens ein Sauerstoff-, Stickstoff- oder Schwefelatom oder eine C-C Doppelbindung oder eine C-C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist.

Die Herstellung der Ausgangsverbindungen erfolgt durch photolytische Monodemethylierung von Rhodosaminyl-Anthracyclinonen in an sich bekannter Weise (Hermentin et al., 4th European Carbohydrate Symposium, Darmstadt, FRG, July 12-17, 1987, Abstracts of Papers, A-144; Hermentin et al., EP 0 270 992 A2). Die Umsetzung zu den erfindungsgemäßen Verbindungen der Formel I erfolgt z.B. durch Reaktion der jeweiligen Ausgangsverbindungen der Formel I mit $R^7$=H mit durch die Definition von $R^7$ vorgegebenen Aldehyden oder Halogeniden. Die Verfahrensbedingungen sind für die Reaktion mit den Aldehyden bekannt (Tong et al., J. Med. Chem. (1979) 22, 912). Die Umsetzung mit Halogeniden erfolgt bevorzugt unter wasserfreien Bedingungen, vorzugsweise in Dimethylformamid oder Acetonitril, bei einer Temperatur von 20° C bis 80° C in Gegenwart einer Base, bevorzugt Triethylamin oder Kaliumcarbonat.

Die nach dem erfindungsgemäßen Verfahren erhaltenen neuen Anthracyclinderivate zeichnen sich durch zytostatische Aktivität aus, und sie können daher zusammen mit den üblichen pharmazeutischen Konfektionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4'-epi-Adriamycin, 4'-Methoxyadriamycin oder 4'-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese

Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des angewandten Testverfahrens ergeben sich aus den nachfolgenden Beschreibungen der Verfahrensweisen zur Ermittlung der Koloniebildung.

Zur Erläuterung des erfindungsgemäßen Herstellungsverfahrens werden nachfolgend Beispiele 1 bis 18 aufgeführt, in denen bevorzugte erfindungsgemäße Verbindungen nach dem beanspruchten Verfahren hergestellt wurden.

Charakterisierung von Verbindungen der Formel I

Der Verlauf der Reaktionen sowie die resultierenden Verbindungen wurden dünnschichtchromatographisch oder mit der HPLC-Technik untersucht. Dünnschichtchromatographie erfolgte, sofern nicht anders vermerkt, auf Kieselgel-Fertigplatten (Merck). Säulenchromatographie erfolgte über Kieselgel 60 (Merck) der Korngröße 0.040-0.063 mm. Die Ausbeuten sind nicht optimiert.

Für die Dünnschicht- und Säulenchromatographie wurden folgende Laufmittelgemische verwendet (Angaben jeweils in Volumenprozent):

| Laufmittelgemische | A | B | C |
|---|---|---|---|
| Zusammensetzung | | | |
| Chloroform (%) | 70 | 89 | 77 |
| Methanol (%) | 18 | 7.4 | 14 |
| Essigsäure (%) | 8.5 | 3 | 7 |
| Wasser (%) | 3.5 | 0.6 | 2 |

Die jeweiligen $R_F$-Werte der hergestellten Verbindungen sind in Tabeile 4 zusammengefaßt.

Die Strukturen der hergestellten Verbindungen wurden mittels $^1$H-NMR- sowie MS-Spektroskopie ermittelt. Die $^1$H-NMR-Daten sind in Tabelle 5 zusammengefaßt.

Beispiele:

Herstellung der Ausgangsverbindungen

Die Herstellung der Ausgangsverbindungen

- 7-0-(3'-N-Methyl-alpha-L-daunosaminyl)-β-rhodomycinon A
  (Verbindung der Formel I mit $R^1 = H$, $R^2 = OH$, $R^3 = R^4 = R^5 = OH$, $R^6 = CH_2CH_3$ und $R^7 = H$),
- 7-0-(3'-N-Methyl-alpha-L-daunosaminyl)-β-isorhodomycinon B
  (Verbindung der Formel I mit $R^1 = R^2 = R^3 = R^4 = R^5 = OH$, $R^6 = CH_2CH_3$ und $R^7 = H$),
- 7-0-(3'-N-Methyl-alpha-L-daunosaminyl)-epsilon-isorhodomycinon C
  (Verbindung der Formel I mit $R^1 = R^2 = R^3 = R^4 = OH$, $R^5$ - $COOCH_3$, $R^6 = CH_2CH_3$ und $R^7 = H$),
- 7-0-(3'-N-Methyl-alpha-L-daunosaminyl)-daunomycinon D
  (Verbindung der Formel I mit $R^1 = H$, $R^2 = OCH_3$, $R^3 = R^4 = OH$, $R^5 = H$, $R^6 = COCH_3$ und $R^7 = H$),
- 7-0-(3'-N-Methyl-alpha-L-daunosaminyl)-adriamycinon E
  (Verbindung der Formel I mit $R^1 = H$, $R^2 = OCH_3$, $R^3 = R^4 = OH$, $R^5 = H$, $R^6 = COCH_2OH$ und $R^7 = H$) und
- 7-0-(3'-N-Methyl-alpha-L-daunosaminyl)-daunomycinon-13-ol F
  (Verbindung der Formel I mit $R^1 = H$, $R^2 = OCH_3$, $R^3 = R^4 = OH$, $R^5 = H$, $R^6 = CHOHCH_3$ und $R^7 = H$)
- 7-0-(3'-N-Methyl-alpha-L-daunosaminyl)-4-0-methyl-β-rhodomycinon G
  (Verbindung der Formel I mit $R^1 = H$, $R^2 = OCH_3$, $R^3 = R^4 = OH$, $R^5 = OH$, $R^6 = CH_2CH_3$ und $R^7 = H$)

erfolgte in an sich bekannter Weise (Hermentin et al., 4th European Carbohydrate Symposium, Darmstadt, FRG, July 12-17, 1987, Abstracts of Papers, A-144; Hermentin et al., EP 0 270 992 A2) durch photolytische Demethylierung der korrespondierenden 7-0-alpha-L-Rhodosaminyl-Anthracyclinone:

- A wurde hergestellt aus β-Rhodomycin I

(7-0-alpha-L-Rhodosaminyl-β-rhodomycinon);

- B wurde hergestellt aus β-Isorhodomycin I (7-0-alpha-L-Rhodosaminyl-β-isorhodomycinon);
- C wurde hergestellt aus 7-0-alpha-L-Rhodosaminyl-epsilon-isorhodomycinon;
- D wurde hergestellt aus N,N-Dimethyl-daunomycin (7-0-alpha-L-Rhodosaminyl-daunomycinon);
- E wurde hergestellt aus N,N-Dimethyl-adriamycin (7-0-alpha-L-Rhodosaminyl-adriamycinon);
- F wurde hergestellt aus N,N-Dimethyl-daunomycin-13-ol (7-0-alpha-L-Rhodosaminyl-daunomycinon-13-ol);
- G wurde hergestellt aus 4-0-Methyl-β-rhodomycin I (4-0-Methyl-7-0-alpha-L-rhodosaminyl-β-rhodomycinon).

Beispiel 1:

7-0-(3'-N -Allyl-3'-N-methyl-alpha-L-daunosaminyl)-β-rhodomycinon (Verbindung 1)

Zu einer Lösung von 7-0-(3'-N-Methyl-alpha-L-daunosaminyl)-β-rhodomycinon (300 mg = 0,567 mmol) und Triethylamin (240 µl = 174 mg = 1,72 mmol = 3,0 equiv.) in 30 ml trockenem Dimethylformamid wurden 75 µl (105 mg = 0,867 mmol = 1,53 equiv.) Allylbromid zugesetzt und bei Raumtemperatur im Dunkeln gerührt. Nach 16 h wurde weiteres Triethylamin (80 µl = 58 mg = 0,574 mmol = 1,0 equiv.) und Allylbromid (25 µl = 35 mg = 0,289 mmol = 0,51 equiv.) zugesetzt und weitere 16 h gerührt. Dann wurde am Rotationsverdampfer im Hochvakuum zur Trockne eingeengt und das Reaktionsgemisch durch zweimalige Säulenchromatographie über Kieselgel (30 g bzw. 20 g) im Laufmittelgemisch C getrennt ($R_F$ 0,49). Die vereinten Fraktionen wurden zur Phasentrennung mit Wasser versetzt, mit 10-proz. (w/v) Natronlauge auf pH 7 gebracht und danach durch Zusatz gesättigter wäßriger Natriumhydrogencarbonat-Lösung auf pH 8 eingestellt. Dann wurden die Phasen im Scheidetrichter getrennt, die wäßrige Phase mehrmals mit Chloroform extrahiert und die vereinigten organischen Phasen am Rotationsverdampfer zur Trockne eingeengt.

Ausbeute: 153 mg (0,27 mmol) = 47 %

Beispiel 2:

7-0-(3'-N -Methyl-3'-N-propargyl-alpha-L-daunosaminyl)-β-rhodomycinon (Verbindung 2)

53 mg (0,10 mmol) 7-0-(3'-N-Methyl-alpha-L-daunosaminyl)-β-rhodomycinon und 106 µl 80 %iges Propargylbromid in Toluol (113 mg = 0,95 mmol = 9,5 equiv.) wurden in Gegenwart von 40 µl (29 mg = 0,287 mmol = 2,87 equiv.) Triethylamin analog Beispiel 1 während 30 min umgesetzt und über 10 g Kieselgel im Laufmittelgemisch B getrennt ($R_F$ 0,29) und aufgearbeitet.

Ausbeute: 31 mg (0,055 mmol) = 55 %

MS-FAB ($M+H^+$) m/e = 568

Beispiel 3:

7-0-(3'-N-Hydroxyethyl-3'-N-methyl-alpha-L-daunosaminyl)-β-rhodomycinon (Verbindung 3 nicht beansprucht)

30 mg (0,057 mmol) 7-0-(3'-N-Methyl-alpha-L-daunosaminyl)-β-rhodomycinon und 50 µl (88 mg = 0,71 mmol = 12,4 equiv.) Bromethanol wurden in Gegenwart von 24 µl (17 mg = 0,17 mmol = 3,0 equiv.) Triethylamin analog Beispiel 1 während 4 Tage umgesetzt und aufgearbeitet. Zur Säulentrennung wurden 15 g Kieselgel mit einer Mischung aus Chloroform/ Methanol (20/1) konditioniert. Dann wurde das flüssige Produktgemisch auf die Säule aufgetragen und das darin enthaltene überschüssige Bromethanol und Dimethylformamid mit Chloroform/Methanol (20/1) (ca. 150 ml) ausgewaschen. Das am Auftragspunkt verbliebene Reaktionsprodukt wurde nachfolgend im Laufmittelgemisch A getrennt ($R_F$ 0,58) und analog Beispiel 1 aufgearbeitet.

Ausbeute: 14 mg (0,024 mmol) = 42 %

Beispiel 4:

7-0-(3'-N-Methyl-3'-N-(4-picolyl)-alpha-L-daunosaminyl)-β-rhodomycinon (Verbindung 4)

Eine Lösung von 100 mg (0,189 mmol) 7-0-(3'-N-Methyl-alpha-L-daunosaminyl)-β-rhodomycinon in 10 ml Acetonitril/Wasser (4/1) wurde mit 50 µl (53 mg = 0,88 mmol = 4,7 equiv.) Essigsäure und 800 mg (713 µl = 7,47 mmol = 39,5 equiv.) Pyridin-4-aldehyd versetzt und 2 h bei Raumtemperatur im Dunkeln gerührt. Dann wurde Natriumcyanoborhydrid (240 mg = 3,82 mmol = 20 equiv.) zugesetzt und die Reaktion weitere 2 h gerührt. Dann wurde die Reaktionslösung in eine wäßrige Natriumhydrogencarbonat-Lösung eingegossen und mit Chloroform extrahiert. Die vereinigten Chloroformphasen wurden mit Wasser vom pH 13 (Zusatz von Natronlauge) erneut extrahiert, wobei die überschüssige Pyridylverbindung im Chloroform und das Anthracyclin (als Natriumsalz; Blaufärbung) im Wasser verblieb. Nach Einstellung der wäßrigen Phase auf pH 8 wurde das Anthracyclin mit Chloroform extrahiert und über 12 g Kieselgel im Laufmittelgemisch B getrennt ($R_F$ 0,23) und analog Beispiel 1 aufgearbeitet.

Ausbeute: 42 mg (0,068 mmol) = 36 %

Beispiel 5:

7-0-(3'-N-Methyl-3'-N-(2-picolyl)-alpha-L-daunosaminyl)-β-rhodomycinon (Verbindung 5)

20 mg (0,038 mmol) 7-0-(3'-N-Methyl-alpha-L-daunosaminyl)-β-rhodomycinon und 160 mg (142 µl = 1,49 mmol = 39 equiv.) Pyridin-2-aldehyd wurden analog Beispiel 4 zur Reaktion gebracht; die säulenchromatographische Trennung erfolgte im Laufmittelgemisch C ($R_F$ 0,79).

Ausbeute: 14 mg (0,023 mmol) = 60 %

Beispiel 6:

7-0-(3'-N-(2-Furfuryl)-3'-N-methyl-alpha-L-daunosaminyl)-β-rhodomycinon (Verbindung 6)

200 mg (0,38 mmol) 7-0-(3'-N-Methyl-alpha-L-daunosaminyl)-β-rhodomycinon und 1,5 ml (1734 mg = 18,0 mmol = 47 equiv.) Furfural wurden analog Beispiel 4 umgesetzt, wobei nach der Natriumcyanoborhydrid-Zugabe 40 h weitergerührt wurde. Dann wurde die Lösung in Wasser eingegossen, mit Natriumhydrogencarbonat auf pH 8 eingestellt, mit Chloroform extrahiert und das Lösungsmittel am Rotationsverdampfer entfernt. Das erhaltene Produktgemisch wurde zur Entfernung überschüssiger Furanylverbindung im Hochvakuum getrocknet und danach über 20 g Kieselgel im Laufmittelgemisch C ($R_F$ 0,57) getrennt und im Laufmittelgemisch B ($R_F$ 0,22) rechromatographiert.

Ausbeute: 129 mg (0,21 mmol) = 55 %

Beispiel 7:

7-0-(3'-N-Acetamido-3'-N-methyl-alpha-L-daunosaminyl)-β-rhodomycinon (Verbindung 7 nicht beansprucht)

30 mg (0,057 mmol) 7-0-(3'-N-Methyl-alpha-L-daunosaminyl)-β-rhodomycinon und 30 mg (0,162 mmol = 2,84 equiv.) Jodacetamid wurden in Gegenwart von 24 µl (17,4 mg = 0,17 mmol = 3,0 equiv.) Triethylamin analog Beispiel 1 während 16 h umgesetzt, wobei jedoch als Lösungsmittel Acetonitril (6 ml) verwendet wurde. Das Produktgemisch wurde über 6 g Kieselgel im Laufmittelgemisch C getrennt ($R_F$ 0,31) und analog Beispiel 1 aufgearbeitet.

Ausbeute: 19 mg (0,032 mmol) = 56 %

Beispiel 8:

7-0-(3'-N-Glycidyl-3'-N-methyl-alpha-L-daunosaminyl)-β-rhodomycinon (Verbindungen 8m, 8a und 8b)

Eine Mischung von 7-0-(3'-N-Methyl-alpha-L-daunosaminyl)-β-rhodomycinon (200 mg = 0,378 mmol), Epibromhydrin (400 µl = 640 mg = 4,675 mmol = 12,4 equiv.) und Kaliumcarbonat (400 mg) in trockenem Acetonitril (25 ml) wurde 30 h bei 60° C im Dunkeln gerührt. Dann wurde am Rotationsverdampfer eingeengt, der Rückstand in Chloroform/Ethanol (20/1) aufgenommen, filtriert und über 20 g Kieselgel in Chloroform/ Ethanol (20/1) getrennt. Dabei wurde laut $^1$H-NMR eine 1:1-Isomerenmischung (Verbindung 8m) in einer Ausbeute von 102 mg (0,17 mmol = 45 %) isoliert.

Rechromatographie in Chloroform/Ethanol (20/1) erlaubte die teilweise Auftrennung des Isomerengemischs 8m in die reinen Isomere 8a ($R_F$ 0,35) und 8b ($R_F$ 0,32).

MS-FAB ($M+H^+$) m/e = 586

Beispiel 9:

7-0-(3'-N-Allyl-3'-N-methyl-alpha-L-daunosaminyl)-β-isorhodomycinon (Verbindung 9)

300 mg (0,55 mmol) 7-0-(3'-N-Methyl-alpha-L-daunosaminyl)-β-isorhodomycinon und 100 µl (140 mg = 1,16 mmol = 21 equiv.) Allylbromid wurden in Gegenwart von 330 µl (240 mg = 2,4 mmol = 44 equiv.) Triethylamin analog Beispiel 1 umgesetzt. Nach 16 h wurden weitere 165 µl (2,2 equiv.) Triethylamin und 50 µl (1 equiv.) Allylbromid zugesetzt und weitere 6 h bei Raumtemperatur im Dunkeln gerührt. Dann wurde im Hochvakuum zur Trockne eingeengt und das Produktgemisch über 52 g Kieselgel im Laufmittel C ($R_F$ 0,52) getrennt. Die gesammelten Fraktionen wurden analog Beispiel 1 aufgearbeitet.

Ausbeute: 154 mg (0,26 mmol) = 48 %

Beispiel 10:

7-0-(3'-N-(Ethoxycarbonylmethyl)-3'-N-methyl-alpha-L-daunosaminyl)-β-rhodomycinon (Verbindung 10 nicht beansprucht)

100 mg (0,189 mmol) 7-0-(3'-N-Methyl-alpha-L-daunosaminyl)-β-rhodomycinon und 75 µl (113 mg = 0,677 mmol = 3,58 equiv.) Bromessigsäureethylester wurden in Gegenwart von 80 µl (58 mg = 0,574 mmol = 3,0 equiv.) Triethylamin analog Beispiel 1 während 2 h umgesetzt. Das Produktgemisch wurde nach der Einengung unverzüglich in wenig Chloroform gelöst, auf eine in Ether angesetzte Kieselgel-Säule (15 g Kieselgel) aufgetragen und zur Entfernung des überschüssigen Bromessigesters mit ca. 100 ml Ether eluiert. Verbindung 10 wurde nachfolgend in Chloroform/Ethanol (20/1) ($R_F$ 0,32) eluiert.

Ausbeute: 70 mg (0,114 mmol) = 60 %

Beispiel 11:

7-0-(3'-N-Carboxymethyl-3'-N-methyl-alpha-L-daunosaminyl)-β-rhodomycinon (Verbindung 11 nicht beansprucht)

20 mg (0,038 mmol) 7-0-(3'-N-Methyl-alpha-L-daunosaminyl)-β-rhodomycinon und 15 µl (29 mg = 0,21 mmol = 5,5 equiv.) Bromessigsäure wurden in Gegenwart von 16 µl (11,6 mg = 0,115 mmol = 3,0 equiv.) Triethylamin analog Beispiel 1 während 2 h umgesetzt und der $R_F$-Wert bestimmt.

Beispiel 12:

7-0-(3'-N-Methyl-3'-N-(3-thenyl)-alpha-L-daunosaminyl)-nyl)-β-rhodomycinon (Verbindung 12)

20 mg (0,038 mmol) 7-0-(3'-N-Methyl-alpha-L-daunosaminyl)-β-rhodomycinon und 156 µl (200 mg = 0,178 mmol = 47 equiv.) Thiophen-3-aldehyd wurden analog Beispiel 4 zur Reaktion gebracht und nach Zusatz des Natriumcyanoborhydrids (48 mg = 0,76 mmol = 20 equiv.) 16 h bei Raumtemperatur weitergerührt. Die säulenchromatographische Trennung erfolgte zunächst in Chloroform (zur Abtrennung überschüssiger Thiophenverbindung) und dann im Laufmittelgemisch B ($R_F$ 0,22).

Ausbeute: 9,2 mg (0,015 mmol) = 39 %

Beispiel 13:

7-0-(3'-N-Methyl-3'-N-(2-thenyl)-alpha-L-daunosaminyl)-β-rhodomycinon (Verbindung 13)

20 mg (0,038 mmol) 7-0-(3'-N-Methyl-alpha-L-daunosaminyl)-β-rhodomycinon und 167 µl (200 mg = 0,178 mmol = 47 equiv.) Thiophen-2-aldehyd wurden analog Beispiel 4 zur Reaktion gebracht und nach Zusatz des Natriumcyanoborhydrids (48 mg = 0,76 mmol = 20 equiv.) zunächst 16 h bei Raumtemperatur und dann weitere 8 h bei 50° C weitergerührt. Die säulenchromatographische Trennung erfolgte über 4 g Kieselgel im Laufmittelgemisch B ($R_F$ 0,25); danach wurde über 3 g Kieselgel im Laufmittelgemisch C ($R_F$ 0,63) rechromatographiert.

Ausbeute: 8,2 mg (0,013 mmol) = 34 %

Beispiel 14:

7-0-(3'-N-Glycidyl-3'-N-methyl-alpha-L-daunosaminyl)-β-isorhodomycinon (Verbindung 14m)

Eine Mischung von 7-0-(3'-N-Methyl-alpha-L-daunosaminyl)-β-isorhodomycinon (85 mg = 0,156 mmol), Epibromhydrin (125 µl = 185 mg = 1,35 mmol = 8,7 equiv.) und Kaliumcarbonat (125 mg) in trockenem Dimethylformamid (8 ml) wurde 3 h bei 70° C im Dunkeln gerührt. Dann wurde am Rotationsverdampfer eingeengt und im Hochvakuum über Nacht getrocknet. Der Rückstand wurde in Wasser gelöst und mit verdünnter Salzsäure auf pH 7,0 eingestellt. Das Produkt wurde mit Chloroform ausgeschüttelt und über 12 g Kieselgel in einem Gemisch aus Laufmittel B und Laufmittel C (1/1) chromatographiert und die gesammelten Fraktionen analog Beispiel 1 aufgearbeitet.

Ausbeute: 68 mg (0,11 mmol) = 70 %

Beispiel 15:

7-0-(3'-N-Allyl-3'-N-methyl-alpha-L-daunosaminyl)-daunomycinon (Verbindung 15)

30 mg (0,055 mmol) 3'-N-Methyl-daunomycin und 10 µl (14 mg = 0,116 mmol = 2,1 equiv.) Allylbromid wurden in Gegenwart von 33 µl (24 mg = 0,24 mmol = 4,4 equiv.) Triethylamin analog Beispiel 1 während 24 h umgesetzt und aufgearbeitet und in Laufmittel C ($R_F$ 0,53) über 5 g Kieselgel getrennt. Die gesammelten Fraktionen wurden analog Beispiel 1 aufgearbeitet.

Ausbeute: 17 mg (0,029 mmol) = 53 %

Beispiel 16:

7-0-(3'-N-Allyl-3'-N-methyl-alpha-L-daunosaminyl)-adriamycinon (Verbindung 16)

32 mg (0,057 mmol) 3'-N-Methyl-adriamycin und 10 µl (14 mg = 0,116 mmol = 2,0 equiv.) Allylbromid wurden in Gegenwart von 33 µl (24 mg = 0,24 mmol = 4,2 equiv.) Triethylamin analog Beispiel 1 während 24 h umgesetzt und aufgearbeitet und in Laufmittel C ($R_F$ 0,22) über 5 g Kieselgel getrennt. Die gesammelten Fraktionen wurden analog Beispiel 1 aufgearbeitet.

Ausbeute: 14 mg (0,023 mmol) = 40 %.

Beispiel 17:

7-0-(3'-N-Allyl-3'-N-methyl-alpha-L-daunosaminyl)-4-0-methyl-β-rhodomycinon (Verbindung 17)

29 mg (0,053 mmol) 7-0-(3'-N-Methyl-alpha-L-daunosaminyl)-4-0-methyl-β-rhodomycinon und 10 µl (14 mg = 0,116 mmol = 2,2 equiv.) Allylbromid wurden in Gegenwart von 33 µl (24 mg = 0,24 mmol = 4,5 equiv.) Triethylamin analog Beispiel 1 während 24 h umgesetzt und aufgearbeitet und in einem Gemisch aus Laufmittel B und Laufmittel C (1/1) über 5 g Kieselgel getrennt. Die gesammelten Fraktionen wurden analog Beispiel 1 aufgearbeitet.

Ausbeute: 18 mg (0,031 mmol) = 58 %.

Beispiel 18:

7-0-(3'-N-Acetonyl-3'-N-methyl-alpha-L-daunosaminyl)-β-rhodomycinon (Verbindung 18 nicht beansprucht)

53 mg (0,10 mmol) 7-0-(3'-N-Methyl-alpha-L-daunosaminyl)-β-rhodomycinon und 0,5 ml (580 mg = 6,27 mmol) Chloraceton wurden in Gegenwart von 1 g Kaliumcarbonat in 10 ml Dimethylformamid 16 h im Dunkeln gerührt. Dann wurde die Lösung abfiltriert und eingeengt und mit Chloroform/ Wasser unter neutralen Bedingungen ausgeschüttelt. Das Produkt der organischen Phase wurde über 10 g Kieselgel in Laufmittel B ($R_F$ 0,16) getrennt.Die gesammelten Fraktionen wurden analog Beispiel 1 aufgearbeitet.

Ausbeute: 27 mg (0,046 mmol) = 46 %.

Zytotoxizität von Verbindungen der Formel I gegen L1210-Leukämiezellen der Maus in vitro

Verfahrensweise zur Ermittlung der Koloniebildung von L1210 Leukämiezellen in Soft-Agar

500 Leukämiezellen pro Platte wurden mit unterschiedlichen Konzentrationen der Testsubstanz 1 Stunde bei 37°

C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37° C inkubiert (5 Vol.-% $CO_2$, 95 % relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in Tabelle 1 zusammengefaßt.

Tabelle 1a1):

| Hergestellte Verbindungen der Formel I | | | | | |
|---|---|---|---|---|---|
| Substanz No. (Beispiel) | $R^1$ | $R^2$ | $R^5$ | $R^6$ | $R^7$ |
| Adriamycin | (Referenz) | | | | |
| 1 | H | OH | OH | $CH_2CH_3$ | $CH_2CHCH_2$ |
| 2 | H | OH | OH | $CH_2CH_3$ | $CH_2CCH$ |
| 3 | H | OH | OH | $CH_2CH_3$ | $CH_2CH_2OH$ |
| 4 | H | OH | OH | $CH_2CH_3$ | 4-picolyl |
| 5 | H | OH | OH | $CH_2CH_3$ | 2-picolyl |
| 6 | H | OH | OH | $CH_2CH_3$ | furfuryl |
| 7 | H | OH | OH | $CH_2CH_3$ | $CH_2CONH_2$ |
| 8m2) | H | OH | OH | $CH_2CH_3$ | glycidyl |
| 8a3) | H | OH | OH | $CH_2CH_3$ | glycidyl-a |
| 8b3) | H | OH | OH | $CH_2CH_3$ | glycidyl-b |
| 9 | OH | OH | OH | $CH_2CH_3$ | $CH_2CHCH_2$ |
| 10 | H | OH | OH | $CH_2CH_3$ | $CH_2COOCH_2CH_3$ |
| 11 | H | OH | OH | $CH_2CH_3$ | $CH_2COOH$ |
| 12 | H | OH | OH | $CH_2CH_3$ | 3-thenyl |
| 13 | H | OH | OH | $CH_2CH_3$ | 2-thenyl |
| 14m2) | OH | OH | OH | $CH_2CH_3$ | glycidyl |
| 15 | H | $OCH_3$ | H | $COCH_3$ | $CH_2CHCH_2$ |
| 16 | H | $OCH_3$ | H | $COCH_2OH$ | $CH_2CHCH_2$ |
| 17 | H | $OCH_3$ | OH | $CH_2CH_3$ | $CH_2CHCH_2$ |
| 18 | H | OH | OH | $CH_2CH_3$ | $CH_2COCH_3$ |

1) für die genannten Verbindungen gilt $R^3 = R^4 = OH$
2) m: Mischung beider Isomere a und b
3) a und b: reine Isomere ohne strukturelle Zuordnung

Tabelle 1b:

| Zytotoxizität der hergestellten Verbindungen der Formel I gegen L 1210 Leukämiezellen in vitro | | |
|---|---|---|
| Substanz No. (Beispiel) | Dauerinkubation $IC_{50}$(µg/ml) | 1 h-Inkubation $IC_{50}$(µg/ml) |
| Adriamycin | 0,02 | 0,04 |
| 1 | 0,01 | 0,08 |
| 2 | 0,08 | 0,3 |
| 3 | 0,02 | 0,044 |
| 4 | 0,023 | 0,1 |
| 5 | 0,038 | 0,24 |
| 6 | 0,03 | 0,038 |
| 7 | 0,084 | 0,24 |

Tabelle 1b: (fortgesetzt)

| Zytotoxizität der hergestellten Verbindungen der Formel I gegen L 1210 Leukämiezellen in vitro | | |
|---|---|---|
| Substanz No. (Beispiel) | Dauerinkubation $IC_{50}$(µg/ml) | 1 h-Inkubation $IC_{50}$(µg/ml) |
| 8m[1)] | 0,002 | 0,0072 |
| 8a[2)] | 0,001 | 0,0034 |
| 8b[2)] | 0,003 | 0,01 |
| 9 | 0,008 | 0,5 |
| 10 | 0,1 | 0,06 |
| 11 | - | - |
| 12 | 0,032 | 0,17 |
| 13 | 0,034 | 0,38 |
| 14m[1)] | 0,005 | 0,0024 |
| 15 | 0,008 | größer 1 |
| 16 | - | - |
| 17 | 0,036 | größer 1 |
| 18 | 0,018 | 0,073 |

[1)] m: Mischung beider Isomere a und b

[2)] a und b: reine Isomere ohne strukturelle Zuordnung

Bestimmung der Kreuzresistenzen in vitro im Vergleich zu Adriamycin

Proliferationstest (MTT-Reduktion)

L1210, A 549 oder HT 29 in exponentieller Wachstumsphase werden in einer Zelldichte von 5 x $10^3$ Zellen/ml in RPMI 1640 in einer 96 Well Mikrotiterplatte für 72 h mit unterschiedlichen Konzentrationen der Testsubstanz bei 37° C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 h Inkubation werden 50 µl einer MTT-Lösung (2,5 mg/ml; MTT = 3-(4,5-Dimethylthiazol-2-yl)-2,5-diphenyl-tetrazoliumbromid)in Phosphat-gepuffertem Kochsalz) zugegeben. In Gegenwart lebender Zellen wird MTT zu einem dunkelroten unlöslichen Formazanfarbstoff reduziert. Diese Reaktion ist nach 7 h (L1210 Zellen) bzw. nach 24 h (A 549, HT 29 Zellen) beendet, und das überstehende Medium wird sorgfältig abgesaugt. Der unlösliche Farbstoff wird durch Zugabe von 100 µl DMSO (Dimethylsulfoxid) aufgelöst und die Extinktion der so entstehenden Lösung anschließend für jedes Well in einem Multiscan Photometer 340 CC der Fa. Flow bei einer Wellenlänge von 492 nm vermessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abtötet, abgelesen werden kann. Für wiederholte Untersuchungen beträgt der Variationskoeffizent weniger als 15 %.

Die Bestimmung der Kreuzresistenz zwischen der jeweiligen Testverbindung und Doxorubicin als Standardverbindung wird mit Hilfe des MTT-Testes (Methodik siehe vorstehend) an sensitiven bzw. resistenten L1210 Leukämiezellen durchgeführt.

Die resistente Zellinie wurde durch Inkubation einer sensitiven Sublinie mit schrittweise ansteigenden Konzentrationen der Referenzverbindung etabliert.

Die $IC_{50}$ der Testverbindung an der resistenten Sublinie bezogen auf die $IC_{50}$ der sensitiven Sublinie ergibt sowohl den Resistenzgrad für die Testverbindung ($DR_{(T)}$) als auch für die Referenzverbindung ($DR_{(R)}$) entsprechend der Formel

$$DR_{T,R} = \frac{IC_{50}\text{ resistente Zellinie}}{IC_{50}\text{ sensitive Zellinie}}$$

Zusätzlich wird der Grad der Kreuzresistenz (DCR) für die Testverbindung berechnet, entsprechend der Formel

$$DCR\ \% = \frac{DR_{(T)} - 1}{DR_{(R)}} \times 100$$

Im Falle, daß der Wirkungsverlust der Testverbindung an der resistenten Linie im Verhältnis zur sensitiven Linie größer ist als derjenige der Referenzverbindung, ist ein Kreuzresistenzgrad von mehr als 100 % möglich.

Die in Tabelle 2 zusammengefaßten Ergebnisse zeigen, daß die bisher überprüften Substanzen 1, 4 und 6 nicht kreuzresistent zu Doxorubicin sind.

Tabelle 2:

| Substanz No. | Testsystem | Inkubationsdauer | Resistenzgrad der Zelle | Kreuzresistenz zu Adriamycin |
|---|---|---|---|---|
| Adriamycin | MTT | 3d | 60-80 | 100,0 |
| 1 | MTT | 3d | 10,0 | 19,0 |
| 4 | MTT | 3d | 1,6 | 0,2 |
| 6 | MTT | 3d | 2,0 | 2,0 |

<u>In vivo-Daten der hergestellten Verbindungen</u>

Ermittlung der orientierenden Toxizität

Zur Ermittlung der orientierenden Toxizität werden NMRI-Mäusen am Tag 0 unterschiedliche Dosen der Testsubstanz, gelöst in 0.5 ml 5 %iger Glucose-Lösung, intraperitoneal injiziert. Kontrollgruppen erhalten lediglich 0.5 ml 5 %ige Glucose-Lösung. Pro Konzentration der Testsubstanz werden 5 Mäuse verwendet. Am Tag 14 wird die Zahl der überlebenden Mäuse ermittelt und daraus nach der Litchfield Wilcoxon Methode die LD5, LD50 und LD95 ermittelt. Die Toxizität (LD50 (mg/kg)) der hier beschriebenen Verbindungen im Vergleich zu Adriamycin ist in Tabelle 3 zusammengefaßt.

In vivo Wirksamkeit von Verbindungen der Formel I gegen L1210 Leukämie der Maus

Methodik:

Ascitesflüssigkeit wird unter sterilen Bedingungen DBA2 Mäusen (weiblich, 18 - 20 g) 7 Tage nach Tumorzell-Inokulation entnommen. Der Ascites wird dreimal mit PBS (Phosphate Buffered Saline) gewaschen, gezählt und auf eine Zellzahl von $10^6$ in 0.2 ml PBS eingestellt.

$10^6$ Zellen, suspendiert in 0.2 ml PBS, werden anschliessend DBF1 Mäusen (weiblich, 18 - 20 g) intraperitoneal injiziert. 6 Tiere pro Gruppe werden für jede Substanzkonzentration bzw. als Kontrolle eingesetzt.

Ermittlung der antitumoralen Wirksamkeit:

a) Die Tiere werden am Tag 1 und 5 nach Injektion der Testsubstanz gewogen. Gewichtsverlust von mehr als 20 % am Tag 5 wird als Indikator einer toxischen Substanzwirkung angesehen.

b) Am Ende des Experimentes (Tod aller Tiere oder überlebende Tiere am Tag 60) wird die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wird ausschließlich für im Verlaufe des Experimentes sterbende Tiere bestimmt. Langzeitüberlebende (LTS) werden bei dieser Berechnung nicht berücksichtigt und gesondert aufgeführt.

Aus der mittleren Überlebenszeit ($MST_T$) der behandelten Gruppen sowie der Kontrollgruppen ($MST_c$) wird die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_T}{MST_c} \times 100$$

Die T/C-Werte und das jeweils eingesetzte Behandlungsschema sind - zusammen mit der orientierenden Toxizität - in Tabelle 3 zusammengefaßt. T/C-Werte größer als 125 % werden als Indikator einer signifikanten antitumoralen Wirksamkeit der Testsubstanz angesehen.

Tabelle 3:

| Wirkung der hergestellten Verbindungen in vivo | | | | |
|---|---|---|---|---|
| Substanz No. (Beispiel) | orientierende Toxizität $LD_{50}$(mg/kg) | | L1210-Leukämie in vivo T/C[3])/Dosis (mg/kg) | |
| | 3xip,q3d[1]) | 3xiv,q3d[2]) | 3xip,q3d[1]) | 3xiv,q3d[2]) |
| 1 | 1-5 | | 200/1,19 | 146/1,00 |
| 2 | | 2-5 | | 144/1,20 |
| 3 | 0,2-1 | | | 108/1,13 |
| 4 | 2,5-5 | | | |
| 6 | 1-5 | | 141/2,80 | 129/2,80 |
| 7 | 2-5 | | | 142/11,2 |
| 8m | 0,75-1,5 | | 114/0,47 | |
| 9 | größer 6 | | | |
| 10 | 10-25 | | | 132/12,7 |
| 12 | | 2-5 | | |
| 14 | | 0,3-0,75 | | 104/0,13 |
| Adriamycin | 2,7 | | 154[4]) | |

1) 3xip,q3d: dreimalige Applikation intraperitoneal im Abstand von jeweils 3 Tagen

2) 3xiv,q3d: dreimalige Applikation intravenös im Abstand von jeweils 3 Tagen

3) T/C: Überlebensrate, ausgedrückt in % der Kontrolle

4) Zwei von 6 Tieren sind geheilt (long term survivors)

Tabelle 4:

| $R_F$-Werte der hergestellten Verbindungen | | | | |
|---|---|---|---|---|
| Substanz No. (Beispiel) | Laufmittelgemisch | | | Chloroform/Ethanol |
| | A | B | C | (20/1) |
| 1 | 0,77 | 0,18 | 0,49 | 0,27 |
| 2 | 0,82 | 0,29 | 0,56 | 0,45 |
| 3 | 0,58 | 0,054 | 0,28 | 0,03 |
| 4 | 0,63 | 0,23 | 0,47 | 0,21 |
| 5 | 0,90 | 0,38 | 0,79 | - |
| 6 | 0,76 | 0,22 | 0,57 | 0,26 |
| 7 | 0,58 | 0,11 | 0,31 | 0,13 |
| 8a | 0,72 | 0,17 | 0,44 | 0,35 |
| 8b | 0,72 | 0,17 | 0,44 | 0,32 |
| 9 | 0,78 | 0,17 | 0,52 | 0,24 |
| 10 | 0,88 | 0,42 | 0,74 | 0,32 |
| 11 | 0,50 | 0,05 | 0,32 | 0 |
| 12 | 0,78 | 0,22 | 0,59 | 0,25 |
| 13 | 0,82 | 0,25 | 0,63 | 0,35 |
| 14a | 0,73 | 0,11 | 0,45 | 0,32 |
| 14b | 0,73 | 0,11 | 0,45 | 0,28 |
| 15 | 0,62 | 0,16 | 0,53 | 0,24 |
| 16 | 0,31 | 0,012 | 0,22 | 0,014 |
| 17 | 0,62 | 0,12 | 0,53 | 0,19 |
| 18 | 0,69 | 0,16 | 0,58 | 0,27 |

<u>Tabelle 5:</u> 300 MHz $^1$H-NMR-Daten verschiedener Verbindungen der Formel I

Die Substanznummern der ersten Zeile entsprechen den jeweiligen Beispielsnummern. Die Messung der Spektren erfolgte, sofern nicht anders vermerkt, in $CDCl_3$ gegen Tetramethylsilan als innerem Standard.

Abkürzungen:
s = Singulett
d = Dublett
t = Triplett
q = Quartett
dd = Dublett eines Dubletts
ddd = Dublett eines Dubletts eines Dubletts
dt = Dublett eines Tripletts
dq = Dublett eines Quartetts
bs = breites Singulett

Tabelle 5, Teil 1:

| Substanz No. | 1 | 2 | 3 | 4 |
|---|---|---|---|---|
| (Proton) | a b c | a b | a b | a b c |
| | $R^7$: $CH_2CHCH_2$ | $CH_2CCH$ | $CH_2CH_2OH$ | $CH_2$– 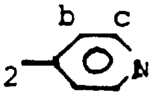 |
| H-1 | 7.89 dd | 7.88 d | 7.88 dd | 7.90 dd |
| H-2 | 7.72 t | 7.72 t | 7.72 t | 7.73 t |
| H-3 | 7.33 dd | 7.33 dd | 7.33 dd | 7.34 dd |
| H-7 | 5.15 m | 5.15 m | 5.15 m | 5.17 m |
| H-8alpha | 2.12 dd | 2.12 dd | 2.12 dd | 2.12 dd |
| H-8ß | 2.26 d | 2.26 d | 2.24 d | 2.27 d |
| H-10 | 4.91 s | 4.91 s | 4.91 s | 4.93 s |
| $H_2$-13 | 1.7-1.9 m | 1.7-1.9 m | 1.7-1.9 m | 1.7-1.9 m |
| $H_3$-14 | 1.12 t | 1.12 t | 1.12 t | 1.13 t |
| H-1' | 5.51 bs | 5.52 d | 5.52 bs | 5.55 bs |
| $H_2$-2' | 1.7-1.9 m | 1.7-1.9 m | 1.7-1.9 m | 1.7-1.9 m |
| H-3' | 2.48 dt | 2.64 ddd | 2.5-2.7 m | 2.62 dt |
| H-4' | 3.70 bs | 3.69 d | 3.76 bs | 3.80 bs |
| H-5' | 4.08 q | 4.09 q | 4.12 q | 4.14 q |
| $H_3$-6' | 1.41 d | 1.41 d | 1.38 d | 1.43 d |
| N-$CH_3$ | 2.18 s | 2.31 s | 2.26 s | 2.12 s |
| OH-4 | 12.15 bs | | | 12.14 bs |
| OH-6 | 12.84 bs | | | 12.86 bs |
| OH-11 | 13.63 bs | | | 13.63 bs |
| a | 2.97dd,<br>3.15dd;<br>$J_{aa'}$=14Hz<br>$J_{ab}=J_{a'b}$=7Hz | 3.39dd,<br>3.41dd;<br>$J_{aa'}$=18Hz<br>$J_{ab}=J_{a'b}$=2Hz | 2.5-2.75m | 3.45d<br>3.62d;<br>$J_{aa'}$=14Hz |
| b | 5.6-5.8m | 2.12t;<br>$J_{ab}=J_{a'b}$=2Hz | 3.61t;<br>J=5Hz | 7.17d;<br>$J_{bc}$=6Hz |
| c | 5.05-5.15m | | | 8.51d;<br>$J_{bc}$=6Hz |

<u>Tabelle 5, Teil 2:</u>

| Substanz No. (Proton) | 5 | 6 | 7[1)] |
|---|---|---|---|
| | a<br>$R_7$:$CH_2$- 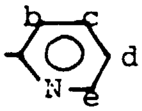 | a<br>$CH_2$- 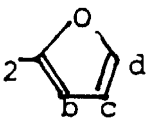 | a b<br>$CH_2CONH_2$ |
| H-1 | 7.89 d | 7.89 d | 7.86 d |
| H-2 | 7.72 t | 7.72 t | 7.72 t |
| H-3 | 7.33 d | 7.33 d | 7.31 d |
| H-7 | 5.17 m | 5.16 m | 5.12 bs |
| H-8alpha | 2.12 dd | 2.12 dd | 2.19 bs |
| H-8ß | 2.28 d | 2.26 d | |
| H-10 | 4.93 s | 4.91 s | 4.83 s |
| $H_2$-13 | 1.7-2.0 m | 1.7-2.0 m | 1.7-1.9 m |
| $H_3$-14 | 1.13 t | 1.12 t | 1.10 t |
| H-1' | 5.55 bs | 5.53 bs | 5.49 bd |
| $H_2$-2' | 1.7-2.0 m | 1.7-1.95 m | 1.97dt[2)];1.7-1.9m |
| H-3' | 2.62 dt | 2.5-2.7 m | 3) |
| H-4' | 3.83 bs | 3.81 bs | 3.72 bs |
| H-5' | 4.15 q | 4.10 q | 4.07 q |
| $H_3$-6' | 1.43 d | 1.42 d | 1.34 d |
| N-$CH_3$ | 2.22 s | 2.22 s | 2.30 s |
| OH-4 | 12.16 bs | 12.15 bs | |
| OH-6 | 12.84 bs | 12.85 bs | |
| OH-11 | 13.61 bs | 13.63 bs | |

Fortsetzung nächste Seite

Fortsetzung Tabelle 5, Teil 2:

| Substanz No. (Proton) | 5 | 6 | 7[1)] |
|---|---|---|---|
| a | 3.59d,3.81d; $J_{aa'}$=14Hz | 3.59d,3.72d; $J_{aa'}$=15Hz | 3.02d,3.09d; $J_{aa'}$=8.5Hz |
| b | 7.24d;$J_{bc}$=8Hz | 6.12d;$J_{bc}$=3Hz | 5.97bs(1H), 7.47bs(1H) |
| c | 7.63ddd; $J_{bc}$=$J_{cd}$=8Hz,$J_{ce}$=2Hz | 6.24dd; $J_{bc}$=3Hz,$J_{cd}$=2Hz | |
| d | 7.15ddd; $J_{cd}$=8Hz,$J_{de}$=5Hz,$J_{bd}$=1Hz | 7.27dd; $J_{cd}$=2Hz,$J_{bd}$=1Hz | |
| e | 8.53dd; $J_{de}$=5Hz,$J_{ce}$=1Hz | | |

1) aufgenommen in $CDCl_3/D_6$-DMSO (5/1)
2) $J_{1',2'}$ = 3 Hz, $J_{2'a,2'b}$ = 12 Hz
3) nicht eindeutig identifiziert

Tabelle 5, Teil 3:

| Substanz No. (Proton) | 8a a $R_7$:$CH_2$- 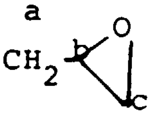 | 8b a $CH_2$- 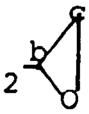 | 9[1] a b c $CH_2CHCH_2$ |
|---|---|---|---|
| H-1 | 7.90 dd | 7.90 dd | 7.29 s, 2H |
| H-2 | 7.73 dd | 7.73 dd | |
| H-3 | 7.34 dd | 7.34 dd | - |
| H-7 | 5.16 m | 5.16 m | 5.0-5.2 m[4] |
| H-8alpha | 2.12 dd | 2.12 dd | 2.20 bs[5] |
| H-8ß | 2.25 d | 2.25 d | |
| H-10 | 4.92 s | 4.91 s | 4.81 s |
| $H_2$-13 | 1.7-1.9 m | 1.7-1.9 m | 1.5-1.7 m |
| $H_3$-14 | 1.12 t | 1.12 t | 1.10 t |
| H-1' | 5.52 t | 5.52 t | 5.48 m |
| $H_2$-2' | 1.7-1.9 m | 1.7-1.9 m | 1.5-1.7 m |
| H-3' | 2) | 2) | 2.49 dt |
| H-4' | 3.69 bs | 3.71 bs | 3.70 bs |
| H-5' | 4.09 q | 4.09 q | 4.08 q |
| $H_3$-6' | 1.40 d | 1.41 d | 1.37 d |
| N-$CH_3$ | 2.31 s | 2.35 s | 2.19 s |
| OH-4 | 12.16 bs | 12.17 bs | 6) |
| OH-6 | 12.85 bs | 12.85 bs | 6) |
| OH-11 | 13.64 bs | 13.63 bs | 6) |
| | 3) | 3) | 7) |

Fortsetzung nächste Seite

Fortsetzung Tabelle 5, Teil 3:

1) aufgenommen in $CDCl_3/D_6$-DMSO (5/1)

2) nicht eindeutig identifiziert

3) die Protonen a-c im Bereich 2,3-3,0 ppm wurden nicht eindeutig zugeordnet

4) überlagert durch $CH_2$ (c)

5) überlagert durch N-$CH_3$

6) phenolische OH bei 12.37 bs (2H) und 13.04 bs (2H)

7) a: 2.99dd (1H), 3.14dd (1H); $J_{aa'}$=14Hz, $J_{ab}$=$J_{a'b}$=6Hz
b: 5.6-5.8m (1H)
c: 5.0-5.2m (überlagert durch H-7)

Tabelle 5, Teil 4:

| Substanz No. (Proton) | 10 | 12 | 13 |
|---|---|---|---|
| | a b c<br>$R^7$: $CH_2COCH_2CH_3$ | a<br>$CH_2$- 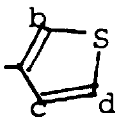 | a<br>$CH_2$- 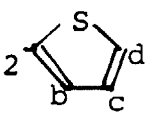 |
| H-1 | 7.90 dd | 7.88 dd | 7.88 dd |
| H-2 | 7.73 t | 7.72 dd | 7.72 t |
| H-3 | 7.33 dd | 7.33 dd | 7.33 dd |
| H-7 | 5.15 m | 5.16 m | 5.16 m |
| H-8alpha | 2.11 dd | 2.13 dd | 2.13 dd |
| H-8ß | 2.26 d | 2.27 d | 2.27 d |
| H-10 | 4.91 s | 4.92 s | 4.92 s |
| $H_2$-13 | 1.7-1.9 m | 1.7-2.0 m | 1.7-2.0 m |
| $H_3$-14 | 1.12 t | 1.13 t | 1.13 t |
| H-1' | 5.51 bd | 5.53 bs | 5.54 bs |
| $H_2$-2' | 1.7-1.9 m | 1.7-2.0 m | 1.7-2.0 m |
| H-3' | 2.82 m | 2.56 m | 2.62 dt |
| H-4' | 3.65 bs | 3.80 bs | 3.79 bs |
| H-5' | 4.09 q | 4.12 q | 4.11 q |
| $H_3$-6' | 1.40 d | 1.43 d | 1.43 d |
| N-$CH_3$ | 2.38 s | 2.12 s | 2.19 s |
| OH-4 | 12.16 bs | 12.14 bs | 12.15 bs |
| OH-6 | 12.84 bs | 12.84 bs | 12.84 bs |
| OH-11 | 13.63 bs | 13.61 bs | 13.62 bs |

Fortsetzung nächste Seite

Fortsetzung Tabelle 5, Teil 4:

| | 10 | 12 | 13 |
|---|---|---|---|
| a | 3.31d, 3.33d; $J_{aa'}$=19Hz | 3.46d, 3.66d; $J_{aa'}$=14Hz | 3.71d, 3.83d; $J_{aa'}$=14Hz |
| b | 4.09q | 7.02dd $J_{bc}$=1Hz, $J_{bd}$=3Hz | 6.82dd $J_{bc}$=3.5Hz, $J_{bd}$=1Hz |
| c | 1.16t | 6.94dd $J_{bc}$=1Hz, $J_{cd}$=5Hz | 6.88dd $J_{bc}$=3.5Hz, $J_{cd}$=5Hz |
| d | | 7.24dd $J_{bd}$=3Hz, $J_{cd}$=5Hz | 7.17dd $J_{bd}$=1Hz, $J_{cd}$=5Hz |

Tabelle 5, Teil 5:

| Substanz No. (Proton) | 14[1)] | 15[3)] |
|---|---|---|
| | a | a b c |
| | R: CH2 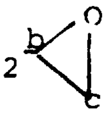 | $CH_2CHCH_2$ |
| H-1 | 7.30 s,2H | 8.04 d |
| H-2 | - | 7.79 t |
| H-3 | | 7.41 d |
| H-7 | 5.13 m | 5.30 m |
| H-8alpha | | 2.10 dd |
| H-8ß | | 2.37 d |
| H-10 | 4.83s | 4) |
| $H_2$-13 | 1.7-2.0 m | - |
| $H_3$-14 | 1.10 t | 2.43 s |
| H-1' | 5.49 bs | 5.57 bd |
| $H_2$-2' | 1.7-2.0 m | 1.84 m |
| H-3' | | 2.57 m |
| H-4' | 3.70 bs | 3.74 bs |
| H-5' | 4.09 q | 4.03 q |
| $H_3$-6' | 1.38 d | 1.38 d |
| $N-CH_3$ | 2.20 s | 2.23 s |
| OH-4 | 2) | - |
| OH-6 | 2) | 13.31 bs |
| OH-11 | 2) | 14.00 bs |

Fortsetzung nächste Seite

Fortsetzung Tabelle 5, Teil 5:

| | 15 |
|---|---|
| a | 3.0-3.25m[5)] |
| b | 5.7-5.9m(1H) |
| c | 5.13bs und<br>5.17d<br>J=3.5Hz |

1) aufgenommen in $CDCl_3$/ $D_6$-DMSO (5/1)

2) phenolische OH bei 12.34bs (2H) und 12.99bs (2H)

3) $OCH_3$ bei 4.10s (3H)

4) 10alpha: 2.98d; 10ß: 3.23d; J=19Hz

5) überlagert durch H-10

Tabelle 5, Teil 6:

| Substanz No. (Proton) | 16 | 17 | 18 |
|---|---|---|---|
| | a b c | a b c | a b |
| | $R^7$ : $CH_2CHCH_2$ | $CH_2CHCH_2$ | $CH_2COCH_3$ |
| H-1 | 8.04 d | 7.89 dd | 7.89 d |
| H-2 | 7.79 t | 7.72 t | 7.72 t |
| H-3 | 7.39 d | 7.33 dd | 7.33 d |
| H-7 | 5.32 m | 5.15 m | 5.14 m |
| H-8alpha | | 2.12 dd | |
| H-8ß | | 2.26 d | |
| H-10 | | 4.91 s | 4.91 s |
| $H_2$-13 | - | 1.7-1.9 m | |
| $H_3$-14 | - | 1.12 t | 1.11 t |
| H-1' | 5.57 bs | 5.51 bs | 5.57 bd |
| $H_2$-2' | | 1.7-1.9 m | |
| H-3' | | 2.48 dt | 2.85 m |
| H-4' | 3.72 bs | 3.70 bs | 3.86 bs |
| H-5' | 4.03 q | 4.08 q | 4.24 q |
| $H_3$-6' | 1.41 | 1.41 d | 1.32 d |
| N-$CH_3$ | 2.22 s | 2.20 s | 2.23 s |
| OH-4 | - | - | 12.16 bs |
| OH-6 | 13.34 bs | 13.36 bs | 12.84 bs |
| OH-11 | 13.98 bs | 13.81 bs | 13.61 bs |
| a | | 2.97 dd, 3.15 dd; $J_{aa'}$= 14 Hz $J_{ab}=J_{a'b}$=7 Hz | 2.37 d, 2.57 d; $J_{aa'}$=11.5 Hz |
| b | 5.68-5.82 m | 5.6-5.8m | 1.42 s, 3H |
| c | 5.1-5.2 m | 5.05-5.15m | |
| $OCH_3$ | 4.09 s | 4.09 s | |

**Patentansprüche**

**Patentansprüche für folgende Vertragsstaaten : AT, BE, CH, DE, FR, GB, IT, LI, LU, NL, SE**

1. Neue zytostatisch wirksame Anthracyclinderivate der allgemeinen Formel I,

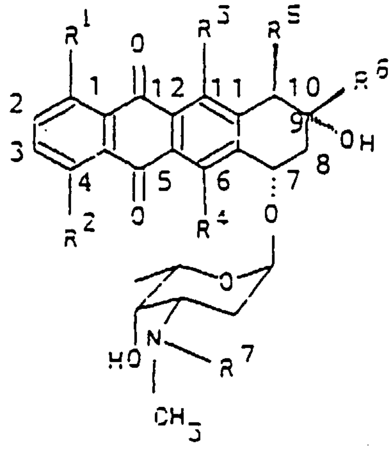

I

welche gegebenenfalls auch als Säureadditionssalz von physiologisch unbedenklichen anorganischen oder organischen Säuren vorliegen, wobei

$R^1$ Wasserstoff oder eine Hydroxygruppe ist,
$R^2$ Wasserstoff, eine Hydroxy- oder eine Methoxygruppe ist,
$R^3$ Wasserstoff oder eine Hydroxygruppe ist,
$R^4$ Wasserstoff oder eine Hydroxygruppe ist,
$R^5$ Wasserstoff, eine Hydroxy- oder eine Methoxycarbonylgruppe ist,
$R^6$ $CH_2CH_3$, $COCH_3$, $COCH_2OH$, $CHOHCH_3$ oder $CHOHCH_2OH$ ist und
$R^7$ ein organischer Substituent mit 2 bis 6 C-Atomen ist, welcher mindestens ein Sauerstoff- oder Stickstoff- oder Schwefelatom oder eine C-C Doppelbindung oder eine C-C Dreifachbindung enthält, wobei die Doppelbindung Bestandteil eines heteroaromatischen Systems sein kann und das Sauerstoff-, Stickstoff- oder Schwefelatom Bestandteil eines heterocyclischen Systems ist.

2. Anthracyclinderivate nach Anspruch 1,
**dadurch gekennzeichnet,**
daß $R^1$ = H, $R^2$ = H, OH oder $OCH_3$, $R^3$ = $R^4$ = OH, $R^5$ = H, und $R^6$ = $COCH_3$, $COCH_2OH$, $CHOHCH_3$ oder $CHOHCH_2OH$ ist.

3. Anthracyclinderivate nach Anspruch 1,
**dadurch gekennzeichnet,**
daß $R^1$ = H oder OH, $R^2$ = $R^3$ = $R^4$ = $R^5$ = OH und $R^6$ = $CH_2CH_3$ ist.

4. Anthracyclinderivate nach Anspruch 1,
**dadurch gekennzeichnet,**
daß $R^1$ = H, $R^2$ = $OCH_3$, $R^3$ = $R^4$ = $R^5$ = OH und $R^6$ = $CH_2CH_3$ ist.

5. Anthracyclinderivate nach Anspruch 1,
**dadurch gekennzeichnet,**
daß $R^1$ = $R^2$ = $R^3$ = $R^4$ = OH, $R^5$ = $COOCH_3$ und $R^6$ = $CH_2CH_3$ ist.

6. Anthracyclinderivate nach Anspruch 1,
**dadurch gekennzeichnet,**
daß $R^7$

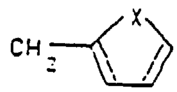 oder 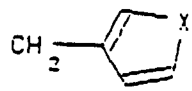

mit X = O, N oder S ist, wobei der Heterocyclus gegebenenfalls durch $-CH_3$, $-NO_2$, $-CH_2OH$, -Cl oder -Br substituiert sein kann, bevorzugt jedoch nicht substituiert ist.

7. Anthracyclinderivate nach Anspruch 1, **dadurch gekennzeichnet,** daß $R^7$ ein 2-Picolyl-, 3-Picolyl- oder 4-Picolyl-, substituiert oder unsubstituiert, und bevorzugt ein 2-Picolyl- oder 4-Picolyl-Rest ist.

8. Anthracyclinderivate nach Anspruch 1, **dadurch gekennzeichnet,** daß $R^7$ ein Allyl-, Crotyl- oder Propargyl- und bevorzugt ein Allyl-Rest ist.

9. Anthracyclinderivate nach Anspruch 1, **dadurch gekennzeichnet,** daß $R^7$ ein Glycidyl-Rest ist.

10. Verfahren zur Herstellung von Anthracyclinderivaten nach Anspruch 1, **dadurch gekennzeichnet,** daß man eine Verbindung der Formel I, in welcher $R^1$ = H oder OH, $R^2$ = H, OH oder $OCH_3$, $R^3$ = H oder OH, $R^4$ = H oder OH, $R^5$ = H, OH oder $COOCH_3$, $R^6$ = $CH_2CH_3$, $COCH_3$, $COCH_2OH$, $CHOHCH_3$ oder $CHOHCH_2OH$ und $R^7$ = H sind, entweder in an sich bekannter Weise in Gegenwart von Natriumcyanoborhydrid mit einem Aldehyd von 2 bis 6 C-Atomen, welcher mindestens ein Sauerstoff-, Stickstoff- oder Schwefelatom oder eine C-C Doppelbindung oder eine C-C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist,
oder in an sich bekannter Weise unter wasserfreien Bedingungen in Gegenwart einer Base mit einer Organo-Halogenverbindung von 2 bis 6 C-Atomen, welche mindestens ein Sauerstoff-, Stickstoff- oder Schwefelatom oder eine C-C Doppelbindung oder eine C-C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist,
zu einer Verbindung der Formel I umsetzt, in welcher $R^1$ bis $R^6$ die angegebene Bedeutung haben und $R^7$ ein organischer Substituent mit 2 bis 6 C-Atomen ist, welcher mindestens ein Sauerstoff-, Stickstoff- oder Schwefelatom oder eine C-C Doppelbindung oder eine C-C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist.

11. Verwendung eines Anthracyclinderivates nach Anspruch 1 in einem Arzneimittel.

**Patentanspruch für folgende Vertragsstaaten : ES, GR**

1. Verfahren zur Herstellung neuer zytostatisch wirksamer Anthracyclinderivate der Formel I,

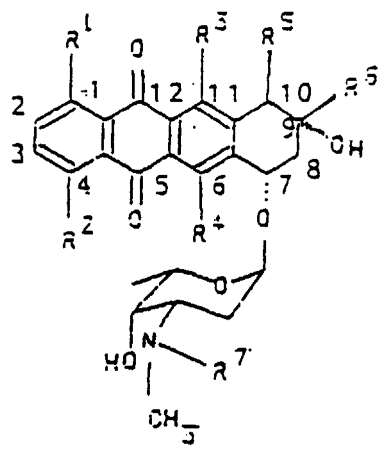

I

welche gegebenenfalls auch als Säureadditionssalz von physiologisch unbedenklichen anorganischen oder organischen Säuren vorliegen, wobei

$R^1$ Wasserstoff oder eine Hydroxygruppe ist,
$R^2$ Wasserstoff, eine Hydroxy- oder eine Methoxygruppe ist,
$R^3$ Wasserstoff oder eine Hydroxygruppe ist,
$R^4$ Wasserstoff oder eine Hydroxygruppe ist,
$R^5$ Wasserstoff, eine Hydroxy- oder eine Methoxycarbonylgruppe ist,
$R^6$ $CH_2CH_3$, $COCH_3$, $COCH_2OH$, $CHOHCH_3$ oder $CHOHCH_2OH$ ist und
$R^7$ ein organischer Substituent mit 2 bis 6 C-Atomen ist, welcher mindestens ein Sauerstoff- oder Stickstoff- oder Schwefelatom oder eine C=C Doppelbindung oder eine C≡C Dreifachbindung enthält, wobei die Doppelbindung Bestandteil eines heteroaromatischen Systems sein kann und das Sauerstoff-, Stickstoff- oder Schwefelatom Bestandteil eines heterocyclischen Systems ist.

**dadurch gekennzeichnet,**
daß man eine Verbindung der Formel I, in welcher $R^1$ = H oder OH, $R^2$ = H, OH oder $OCH_3$, $R^3$ = H oder OH, $R^4$ = H oder OH, $R^5$ = H, OH oder $COOCH_3$, $R^6$ = = $CH_2CH_3$, $COCH_3$, $COCH_2OH$, $CHOHCH_3$ oder $CHOHCH_2OH$ und $R^7$ = H sind, entweder in an sich bekannter Weise in Gegenwart von Natriumcyanoborhydrid mit einem Aldehyd von 2 bis 6 C-Atomen, welcher mindestens ein Sauerstoff-, Stickstoff- oder Schwefelatom oder eine C=C Doppelbindung oder eine C≡C Dreifachbindung enthält, wobei die Doppelbindung auch Bestandteil eines heteroaromatischen Systems sein kann und das Sauerstoff-, Stickstoff- oder Schwefelatom Bestandteil eines offenkettigen oder heterocyclischen Systems ist,
oder in an sich bekannter Weise unter wasserfreien Bedingungen in Gegenwart einer Base mit einer Organo-Halogenverbindung von 2 bis 6 C-Atomen, welche mindestens ein Sauerstoff-, Stickstoff- oder Schwefelatom oder eine C=C Doppelbindung oder eine C≡C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist, zu einer Verbindung der Formel I umsetzt, in welcher $R^1$ bis $R^6$ die angegebene Bedeutung haben und $R^7$ ein organischer Substituent mit 2 bis 6 C-Atomen ist, welcher mindestens ein Sauerstoff-, Stickstoff- oder Schwefelatom oder eine C=C Doppelbindung oder eine C≡C Dreifachbindung enthält, wobei die Doppelbindung auch Bestandteil eines heteroaromatischen Systems sein kann und das Sauerstoff-, Stickstoff- oder Schwefelatom Bestandteil eines heterocyclischen Systems ist, und das Umsetzungsprodukt gegebenenfalls mittels einer physiologisch unbedenklichen anorganischen oder organischen Säure in ein Säureadditionssalz umwandelt.

**Claims**

**Claims for the following Contracting States : AT, BE, CH, DE, FR, GB, IT, LI, LU, NL, SE**

**1.** New anthracycline derivatives having cytostatic activity and the general formula I

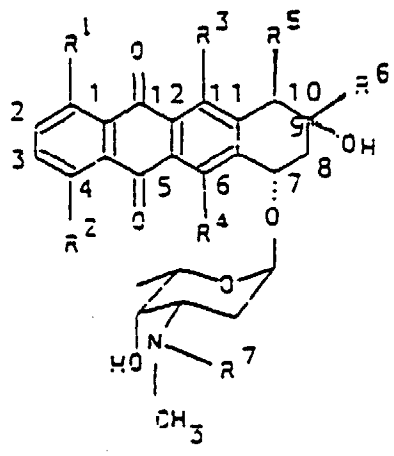

I

which are optionally also in the form of an acid addition salt of physiologically acceptable inorganic or organic acids, where

$R^1$ is hydrogen or a hydroxyl group,
$R^2$ is hydrogen, a hydroxyl or a methoxy group,
$R^3$ is hydrogen or a hydroxyl group,
$R^4$ is hydrogen or a hydroxyl group,
$R^5$ is hydrogen, a hydroxyl or a methoxycarbonyl group,
$R^6$ is $CH_2CH_3$, $COCH_3$, $COCH_2OH$, $CHOHCH_3$ or $CHOHCH_2OH$ and
$R^7$ is an organic substituent which has 2 to 6 carbon atoms and which contains at least one oxygen or nitrogen or sulfur atom or a C-C double bond or a C-C triple bond, it being possible for the double bond to be a constituent of a heteroaromatic system, and the oxygen, nitrogen or sulfur atom being a constituent of a heterocyclic system.

**2.** Anthracycline derivatives as claimed in claim 1, wherein $R^1$ = H, $R^2$ = H, OH or $OCH_3$, $R^3$ = $R^4$ = OH, $R^5$ = H, and $R^6$ = $COCH_3$, $COCH_2OH$, $CHOHCH_3$ or $CHOHCH_2OH$.

**3.** Anthracycline derivatives as claimed in claim 1, wherein $R^1$ = H or OH, $R^2$ = $R^3$ = $R^4$ = $R^5$ = OH and $R^6$ = $CH_2CH_3$.

**4.** Anthracycline derivatives as claimed in claim 1, wherein $R^1$ = H, $R^2$ = $OCH_3$, $R^3$ = $R^4$ = $R^5$ = OH and $R^6$ = $CH_2CH_3$.

**5.** Anthracycline derivatives as claimed in claim 1, wherein $R^1$ = $R^2$ = $R^3$ = $R^4$ = OH, $R^5$ = $COOCH_3$ and $R^6$ = $CH_2CH_3$.

**6.** Anthracycline derivatives as claimed in claim 1, wherein $R^7$ is

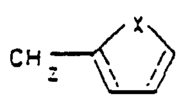 or 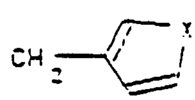

with X = O, N or S, it being possible for the heterocycle optionally to be substituted by $-CH_3$, $-NO_2$, $-CH_2OH$, -Cl or -Br, but being preferably unsubstituted.

**7.** Anthracycline derivatives as claimed in claim 1, wherein $R^7$ is a 2-picolyl, 3-picolyl or 4-picolyl radical, substituted or unsubstituted, and is preferably a 2-picolyl or 4-picolyl radical.

**8.** Anthracycline derivatives as claimed in claim 1, wherein $R^7$ is an allyl, crotyl or propargyl, and preferably an allyl, radical.

**9.** Anthracycline derivatives as claimed in claim 1, wherein $R^7$ is a glycidyl radical.

**10.** A process for the preparation of anthracycline derivatives as claimed in claim 1, which comprises reacting a compound of the formula I in which $R^1$ = H or OH, $R^2$ = H, OH or $OCH_3$, $R^3$ = H or OH, $R^4$ = H or OH, $R^5$ = H, OH or $COOCH_3$, $R^6$ = $CH_2CH_3$, $COCH_3$, $COCH_2OH$, $CHOHCH_3$ or $CHOHCH_2OH$ and $R^7$ = H, either in a manner known per se in the presence of sodium cyanoborohydride with an aldehyde which is of 2 to 6 carbon atoms and which contains at least one oxygen, nitrogen or sulfur atom or a C-C double bond or a C-C triple bond, it being possible for the double bond also to be a constituent of a heteroaromatic system, and the oxygen, nitrogen or sulfur atom being a constituent of a heterocyclic system, or in a manner known per se, under anhydrous conditions in the presence of a base, with an organohalogen compound which is of 2 to 6 carbon atoms and which contains at least one oxygen, nitrogen or sulfur atom or a C-C double bond or a C-C triple bond, it being possible for the double bond also to be a constituent of a heteroaromatic system, and the oxygen, nitrogen or sulfur atom being a constituent of a heterocyclic system, to give a compound of the formula I in which $R^1$ to $R^6$ have the stated meaning and $R^7$ is an organic substituent which has 2 to 6 carbon atoms and which contains at least one oxygen, nitrogen or sulfur atom or a C-C double bond or a C-C triple bond, it being possible for the double bond also to be a constituent of a heteroaromatic system, and the oxygen, nitrogen or sulfur atom being a constituent of a heterocyclic system.

**11.** The use of an anthracycline derivative as claimed in claim 1 in a pharmaceutical.

**Claim for the following Contracting States : ES, GR**

**1.** A process for the preparation of new anthracycline derivatives having cytostatic activity and the general formula I

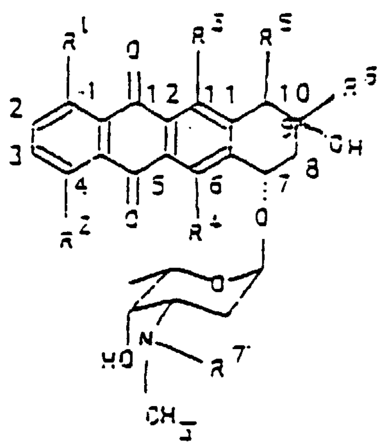

I

which are optionally also in the form of an acid addition salt of physiologically acceptable inorganic or organic acids, where

- $R^1$ is hydrogen or a hydroxyl group,
- $R^2$ is hydrogen, a hydroxyl or a methoxy group,
- $R^3$ is hydrogen or a hydroxyl group,
- $R^4$ is hydrogen or a hydroxyl group,
- $R^5$ is hydrogen, a hydroxyl or a methoxycarbonyl group,
- $R^6$ is $CH_2CH_3$, $COCH_3$, $COCH_2OH$, $CHOHCH_3$ or $CHOHCH_2OH$ and
- $R^7$ is an organic substituent which has 2 to 6 carbon atoms and which contains at least one oxygen or nitrogen or sulfur atom or a C=C double bond or a C≡C triple bond, it being possible for the double bond to be a constituent of a heteroaromatic system, and the oxygen, nitrogen or sulfur atom being a constituent of a heterocyclic system,

which comprises reacting a compound of the formula I in which $R^1$ = H or OH, $R^2$ = H, OH or $OCH_3$, $R^3$ = H or OH, $R^4$ = H or OH, $R^5$ = H, OH or $COOCH_3$, $R^6$ = $CH_2CH_3$, $COCH_3$, $COCH_2OH$, $CHOHCH_3$ or $CHOHCH_2OH$ and $R^7$ = H, either in a manner known per se in the presence of sodium cyanoborohydride with an aldehyde which is of 2 to 6 carbon atoms and which contains at least one oxygen, nitrogen or sulfur atom or a C=C double bond or a C≡C triple bond, it being possible for the double bond also to be a constituent of a heteroaromatic system, and the oxygen, nitrogen or sulfur atom being a constituent of an open-chain or heterocyclic system, or in a manner known per se, under anhydrous conditions in the presence of a base, with an organohalogen compound which is

of 2 to 6 carbon atoms and which contains at least one oxygen, nitrogen or sulfur atom or a C=C double bond or a C≡C triple bond, it being possible for the double bond also to be a constituent of a heteroaromatic system, and the oxygen, nitrogen or sulfur atom being a constituent of a heterocyclic system, to give a compound of the formula I in which $R^1$ to $R^6$ have the stated meaning and $R^7$ is an organic substituent which has 2 to 6 carbon atoms and which contains at least one oxygen, nitrogen or sulfur atom or a C=C double bond or a C≡C triple bond, it being possible for the double bond also to be a constituent of a heteroaromatic system, and the oxygen, nitrogen or sulfur atom being a constituent of a heterocyclic system, and converting the reaction product into an acid addition salt optionally by means of a physiologically acceptable inorganic or organic acid.

## Revendications

### Revendications pour les Etats contractants suivants : AT, BE, CH, DE, FR, GB, IT, LI, LU, NL, SE

**1.** Nouveaux dérivés d'anthracycline à activité cytostatique, de formule générale I

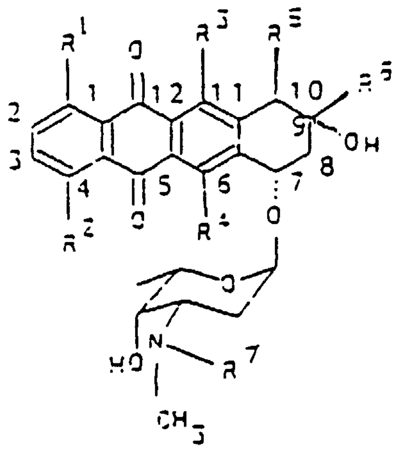

I

qui éventuellement se trouvent également sous forme de sel d'addition avec un acide formé avec des acides organiques ou minéraux physiologiquement acceptables, formule dans laquelle

$R^1$ est un atome d'hydrogène ou le groupe hydroxyle,
$R^2$ est un atome d'hydrogène ou le groupe hydroxyle ou méthoxy,
$R^3$ est un atome d'hydrogène ou le groupe hydroxyle,
$R^4$ est un atome d'hydrogène ou le groupe hydroxyle,
$R^5$ est un atome d'hydrogène ou le groupe hydroxyle ou méthoxycarbonyle,
$R^6$ est $CH_2CH_3$, $COCH_3$, $COCH_2OH$, $CHOHCH_3$ ou $CHOHCH_2OH$ et
$R^7$ est un substituant organique ayant de 2 à 6 atomes de carbone, qui comporte au moins un atome d'oxygène, d'azote ou de soufre ou une double liaison C-C ou une triple liaison C-C, la double liaison pouvant faire partie d'un système hétéroaromatique et l'atome d'oxygène, d'azote ou de soufre faisant partie d'un système hétérocyclique.

**2.** Dérivés d'anthracycline selon la revendication 1, caractérisés en ce que $R^1$ = H, $R^2$ = H, OH ou $OCH_3$, $R^3$ = $R^4$ = OH, $R^5$ = H et $R^6$ = $COCH_3$, $COCH_2OH$, $CHOHCH_3$ ou $CHOHCH_2OH$.

**3.** Dérivés d'anthracycline selon la revendication 1, caractérisés en ce que $R^1$ = H ou OH, $R^2$ = $R^3$ = $R^4$ = $R^5$ = OH, et $R^6$ = $CH_2CH_3$.

**4.** Dérivés d'anthracycline selon la revendication 1, caractérisés en ce que $R^1$ = H, $R^2$ = $OCH_3$, $R^3$ = $R^4$ = $R^5$ = OH, et $R^6$ = $CH_2CH_3$.

**5.** Dérivés d'anthracycline selon la revendication 1, caractérisés en ce que $R^1$ = $R^2$ = $R^3$ = $R^4$ = OH, $R^5$ = $COOCH_3$ et $R^6$ = $CH_2CH_3$.

**6.** Dérivés d'anthracycline selon la revendication 1, caractérisés en ce que $R^7$ représente

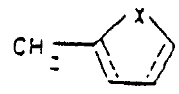

ou

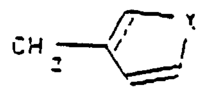

avec X = O, N ou S, l'hétérocycle pouvant éventuellement être substitué par $-CH_3$, $-NO_2$, $-CH_2OH$, -Cl ou -Br, mais de préférence n'est pas substitué.

**7.** Dérivés d'anthracycline selon la revendication 1, caractérisés en ce que $R^7$ est un radical 2-picolyle, 3-picolyle ou 4-picolyle, substitué ou non substitué, et de préférence le radical 2-picolyle ou 4-picolyle,

**8.** Dérivés d'anthracycline selon la revendication 1, caractérisés en ce que $R^7$ est un radical allyle, crotyle ou propargyle, et de préférence le radical allyle.

**9.** Dérivés d'anthracycline selon la revendication 1, caractérisés en ce que $R^7$ est le radical glycidyle.

**10.** Procédé pour la préparation des dérivés d'anthracycline selon la revendication 1, dans lequel on fait réagir un composé de formule I, dans lequel $R^1$ = H ou OH, $R^2$ = H, OH ou $OCH_3$, $R^3$ = H ou OH, $R^4$ = H ou OH, $R^5$ = H, OH ou $COOCH_3$, $R^6$ = $CH_2CH_3$, $COCH_3$, $COCH_2OH$, $CHOHCH_3$ ou $CHOHCH_2OH$ et $R^7$ = H, soit d'une façon connue en soi, en présence de cyanoborohydrure de sodium, avec un aldéhyde ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique, ou, d'une façon connue en soi, dans des conditions anhydres, en présence d'une base, avec un composé organohalogéné ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique, pour aboutir à un composé de formule I dans lequel $R^1$ à $R^6$ ont les significations indiquées et $R^7$ est un substituant organique ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique.

**11.** Utilisation d'un dérivé d'anthracycline selon la revendication 1, dans un médicament.

**Revendication pour les Etats contractants suivants : ES, GR**

**1.** Procédé pour la préparation de nouveaux dérivés d'anthracycline à activité cytostatique, de formule I

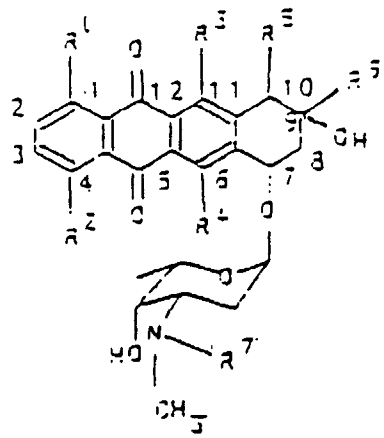

I

qui éventuellement se trouvent également sous forme de sel d'addition avec un acide formé avec des acides organiques ou minéraux physiologiquement acceptables, formule dans laquelle

- $R^1$ est un atome d'hydrogène ou le groupe hydroxyle,
- $R^2$ est un atome d'hydrogène ou le groupe hydroxyle ou méthoxy,
- $R^3$ est un atome d'hydrogène ou le groupe hydroxyle,
- $R^4$ est un atome d'hydrogène ou le groupe hydroxyle,
- $R^5$ est un atome d'hydrogène ou le groupe hydroxyle ou méthoxycarbonyle,
- $R^6$ est $CH_2CH_3$, $COCH_3$, $COCH_2OH$, $CHOHCH_3$ ou $CHOHCH_2OH$ et
- $R^7$ est un substituant organique ayant de 2 à 6 atomes de carbone, qui comporte au moins un atome d'oxygène, d'azote ou de soufre ou une double liaison C-C ou une triple liaison C-C, la double liaison pouvant faire partie d'un système hétéroaromatique et l'atome d'oxygène, d'azote ou de soufre faisant partie d'un système hétérocyclique,

caractérisé en ce qu'on fait réagir un composé de formule I, dans lequel $R^1$ = H ou OH, $R^2$ = H, OH ou $OCH_3$, $R^3$ = H ou OH, $R^4$ = H ou OH, $R^5$ = H, OH ou $COOCH_3$, $R^6$ = $CH_2CH_3$, $COCH_3$, $COCH_2OH$, $CHOHCH_3$ ou $CHOHCH_2$ et $R^7$ = H, soit d'une façon connue en soi, en présence de cyanoborohydrure de sodium, avec un aldéhyde ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique, ou, d'une façon connue en soi, dans des conditions anhydres, en présence d'une base, avec un composé organohalogéné ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique, pour aboutir à un composé de formule I dans lequel $R^1$ à $R^6$ ont les significations indiquées et $R^7$ est un substituant organique ayant de 2 à 6 atomes de carbone, qui comporte au moins un atome d'oxygène, d'azote ou de soufre ou une double liaison C-C ou une triple liaison C-C, la double liaison pouvant également faire partie d'un système hétéroaromatique et l'atome d'oxygène, d'azote ou de soufre faisant partie d'un système hétérocyclique, et éventuellement on convertit le produit de réaction en un sel d'addition avec un acide, au moyen d'un acide organique ou minéral physiologiquement acceptable.